# EUROPEAN PATENT SPECIFICATION

(11) **EP 2 714 085 B1**
(45) Date of publication and mention of the grant of the patent: **01.06.2016**
(21) Application number: 12715096.9
(22) Date of filing: 17.04.2012
(51) Int. Cl.: A61K 36/63, A61K 31/353, A61K 31/7048

(54) **ANTI-INFLAMMATORY COMPOSITION**
ENTZÜNDUNGSHEMMENDE ZUSAMMENSETZUNG
COMPOSITION ANTI-INFLAMMATOIRE

(30) Priority: 27.05.2011 EP 11167804
(43) Date of publication of application: 09.04.2014
(73) Proprietor: Unilever PLC, London EC4Y 0DY (GB); Unilever N.V., 3013 AL Rotterdam (NL)
(72) Inventor: BARRETT, Karen, Elizabeth, Bedford Bedfordshire MK44 1LQ (GB); JENKINS, Gail, Bedford Bedfordshire MK44 1LQ (GB); LOTITO, Silvina, Beatriz, Bedford Bedfordshire MK44 1LQ (GB); WAINWRIGHT, Linda, Jane, Bedford Bedfordshire MK44 1LQ (GB)
(74) Representative: Warner, Guy Jonathan
(86) International application number: PCT/EP2012/056981
(87) International publication number: WO 2012/163590

(56) References cited:
- EP-A1- 1 852 430
- WO-A1-97/32947
- WO-A1-2005/072726
- WO-A1-2006/087748
- WO-A1-2010/070183
- MANNA C; MIGLIARDI V; GOLINO P; SCOGNAMIGLIO A; GALLETTI P; CHIARIELLO M; ZAPPIA V: "Oleuropein prevents oxidative myocardial injury induced by ischemia and reperfusion.", THE JOURNAL OF NUTRITIONAL BIOCHEMISTRY AUG 2004 LNKD- PUBMED:15302080, vol. 15, no. 8, August 2004 (2004-08), pages 461-466, XP002654432, ISSN: 0955-2863
- BAGCHI D; SEN C K; RAY S D; DAS D K; BAGCHI M; PREUSS H G; VINSON J A: "Molecular mechanisms of cardioprotection by a novel grape seed proanthocyanidin extract.", MUTATION RESEARCH, vol. 523-524, no. Special Issue, February 2003 (2003-02), pages 87-97, XP002654433, ISSN: 0027-5107

## Description

This invention relates to the provision of an oral anti-inflammatory composition, in particular to an oral composition for the treatment or prevention of cardiovascular disease and the complications of hypertension.

Cardiovascular disease (a class of diseases that involve the heart or blood vessels) contributes to over a third of global deaths and is currently the leading cause of death in developing countries. The risk of heart disease increases as you age and this term technically refers to any disease that affects the cardiovascular system. It is usually used to refer to those related to atherosclerosis (arterial disease).

Libby (American J. Clinical Nutrition, 83(2), 456S-460S (2006)) discloses that several pro-inflammatory markers, such as IL-6, associate with cardiovascular disease. IL-6 is a cytokine, or cell-signalling protein, encoded in humans by the IL-6 gene which is both pro-inflammatory and anti-inflammatory and which stimulates the immune response to trauma leading to inflammation. Libby et al. (Circulation, 105, 1135-1143 (2002)) discloses that epidemiological studies have found increased vascular risk in association with increased basal levels of cytokines such as IL-6. He further discloses that adipose tissue can synthesize cytokines such as IL-6 so that obesity itself appears to promote inflammation. Libby et al. also mentions that inflammation may participate in hypertension providing a pathophysiological link to atherosclerosis. Thus the identification of actives that down-regulate inflammatory markers, such as IL-6, may provide a route to meeting the continuing need for effective oral compositions for the treatment or prevention of cardiovascular disease and the complications of hypertension.

EP 1 852 430 A discloses a composition containing as its main component proanthocyanidin oligomer to which a substance having a phloroglucinol ring structure or resorcinol ring structure has been bonded, which is obtainable by heating, for example at 90 to 100 degrees centigrade for 1 to 4 hours, plant materials comprising proanthocyanidin polymer or aqueous extract thereof comprising proanthocyanidin polymer, with a substance having a phloroglucinol ring structure or resorcinol ring structure in an acidic aqueous solution. The resulting mixture may then be filtered, concentrated, fractionated or dried using standard techniques known to the skilled person. A best mode for preparing the composition is disclosed in paragraphs [0015] to [0023] of EP 1 852 430 A and is incorporated herein by reference. Such a composition has been reduced in molecular weight compared to the proanthocyanidin polymer feed stock to such a level that the oligomer can be more easily absorbed into a living body. The composition comprises compounds of structure (I): 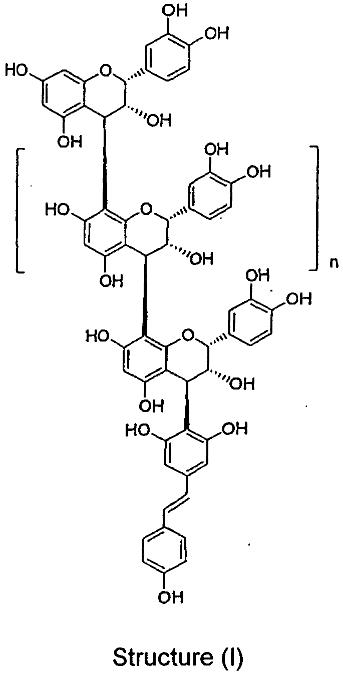 wherin n is 0 or an integer of 1 or 2, and/or structure (II): 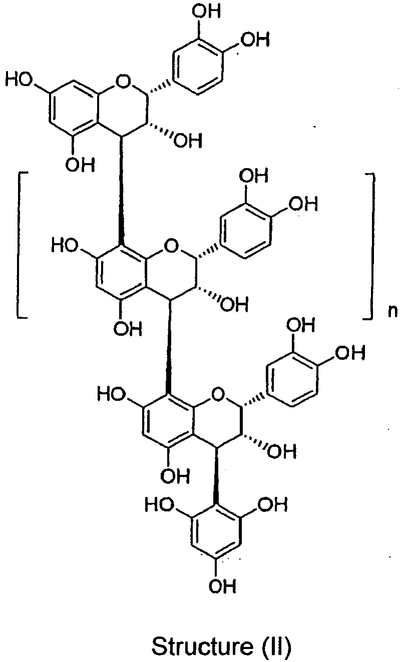 wherin n is 0 or an integer of 1 or 2. Oligomers of structure (I) and (II) are obtained by fractionating the aforementioned composition of EP 1 852 430 A using standard fractionation methods known to the skilled person in the art.

The plant material comprising proanthocyanidin polymer or extract thereof comprising proanthocyanidin polymer may be selected from the group consisting of grape, pine, Chamaecyparis obtuse, camphor tree, wax myrtle, cacao, date plum, banana, Chinese quince, apple, hawthorn, Lychi chinensis, Myrica rubra and Cinnamomi cortex. The substance having a phloroglucinol ring structure or resorcinol ring structure may be selected from the group consisting of resveratrol, phloroglucinol, flavonoid and flavanoid. The substance having a phloroglucinol ring structure or resorcinol ring structure may also be selected from the group consisting of green tea, fresh tea leaves, grape seed, grape seed coat, cube gambir, red algae and extracts thereof.

EP 1 852 430 A further discloses that such a composition exhibits significant antioxidative activity in a 1,1-diphenyl-2-picrylhydrazyl (DPPH) radical-scavenging activity assay and a Trolox Equivalent Antioxidant Capacity (TEAC) assay. It is also disclosed that application of such compositions leads to improved cell viability under ultra-violet radiation, significant anti-oxidant activity to blood serum and liver lipid peroxides (in mice) and reductions in blood serum levels of the liver enzymes glutamyl oxaloacetic transaminase (GOT) and glutamyl pyruvic transaminase (GPT). GOT and GPT are involved in transferring the amino group of amino acids from alpha-amino acids to alpha-keto acids. Transaminases are stored in the liver and are normally present only at low levels in blood serum. They are released into blood when the liver cells are damaged, thus detection of high levels of transaminases in blood serum is evidence of liver cell damage.

A composition according to EP 1 852 430 A is available commercially under the name Oligonol™ (Cognis/BASF)) and comprises proanthocyanidin oligomer to which a substance having a phloroglucinol ring structure or resorcinol ring structure has been bonded, which is obtainable by heating plant materials comprising proanthocyanidin polymer or extract thereof comprising proanthocyanidin polymer, with a substance having a phloroglucinol ring structure or resorcinol ring structure in an acidic aqueous solution., the plant material being an extract produced from a combination of fruit from Lychi chinensis and green leaf of Camellia sinensis.

Aquasol™ (Sabinsa) is a 90:10 v/v ethanol:demineralised water extract of the leaves of Olea europaea, the extract comprising 2.5 % w/v oleuropein, 1.9 % w/v hydroxytyrosol. Tyrosol does not seem to be present. The structures are given hereinbelow:

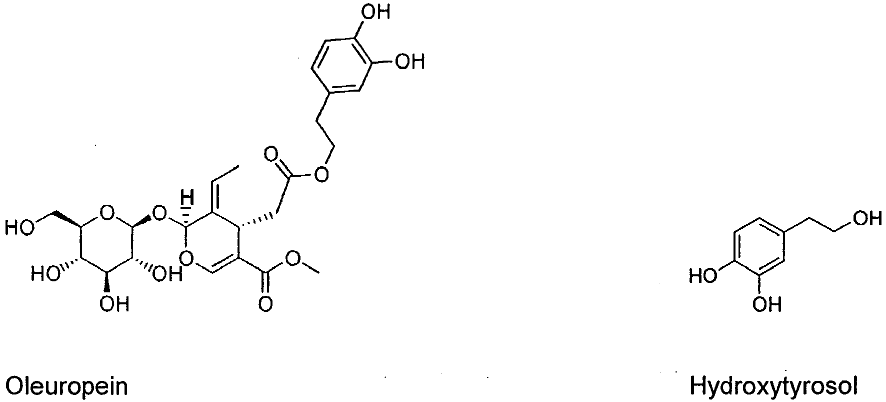

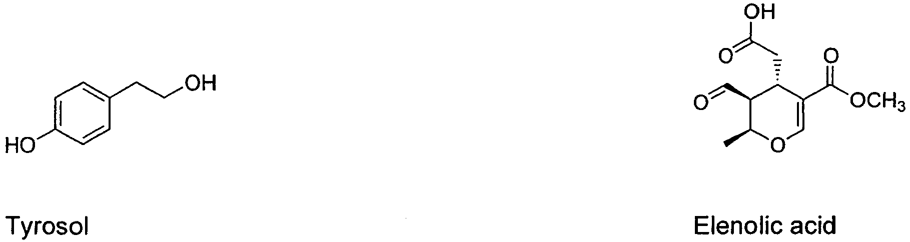

Hydroxytyrosol is one of the main phenolic compounds in Olea europaea, virgin olive oil and waste water obtained from the virgin olive oil production. In freshly produced virgin olive oil, hydroxytyrosol is mainly found in an elenolic ester form (known as oleuropein) which, over time, hydrolyses to the non-esterified form.

Aquasol™ is prepared from the aforementioned extract which is then concentrated under vacuum to 30 % total dry solids and then spray dried.

The inventors have observed that a combination of Oligonol™ and Aquasol™ exhibits a synergistic anti-inflammatory effect.

### Summary of the invention

Thus in a first aspect of the invention, an oral composition for preventing or treating cardiovascular disease or the complications of hypertension is provided, the composition comprising an aqueous ethanol extract of Olea europaea or oleuropein, and a composition containing as its main component proanthocyanidin oligomer to which a substance having a phloroglucinol ring structure or resorcinol ring structure has been bonded, which is obtainable by heating plant materials comprising proanthocyanidin polymer or aqueous extract thereof comprising proanthocyanidin polymer, with a substance having a phloroglucinol ring structure or resorcinol ring structure in an acidic aqueous solution, obtainable by heating plant material being an extract produced from a combination of fruit from Lychi chinensis and green leaf of Camellia sinensis in an acidic aqueous solution is Oligonol™; and wherein the aqueous ethanol extract of Olea europaea is a 90:10 v/v ethanol:demineralised water extract of the leaves of Olea europaea, the extract comprising 2.5 % w/v oleuropein and 1.9 % w/v hydroxytyrosol.

The composition may comprise 10-5000, preferably 50-2500, most preferably 100-300 mg Olea europaea and 10-5000, preferably 50-2500, most preferably 100-300 mg composition containing as its main component proanthocyanidin oligomer to which a substance having a phloroglucinol ring structure or resorcinol ring structure has been bonded, which is obtainable by heating plant materials comprising proanthocyanidin polymer or aqueous extract thereof comprising proanthocyanidin polymer, with a substance having a phloroglucinol ring structure or resorcinol ring structure in an acidic aqueous solution.

The weight ratio of aqueous ethanol extract of Olea europaea to composition containing as its main component proanthocyanidin oligomer to which a substance having a phloroglucinol ring structure or resorcinol ring structure has been bonded, which is obtainable by heating plant materials comprising proanthocyanidin polymer or aqueous extract thereof comprising proanthocyanidin polymer, with a substance having a phloroglucinol ring structure or resorcinol ring structure in an acidic aqueous solution may be 10:1 to 1000:1, preferably 10:1 to 100:1.

The proanthocyanidin oligomer may have a degree of polymerisation of 2 to 4.

The plant material comprising proanthocyanidin polymer or aqueous extract thereof comprising proanthocyanidin polymer may be selected from the group consisting of grape, pine, Chamaecyparis obtuse, camphor tree, wax myrtle, cacao, date plum, banana, Chinese quince, apple, hawthorn, Lychi chinensis, Myrica rubra and Cinnamomi cortex.

The substance having a phloroglucinol ring structure or resorcinol ring structure may be selected from the group consisting of resveratrol, phloroglucinol, flavonoid and flavanoid.

The substance having a phloroglucinol ring structure or resorcinol ring structure may be selected from the group consisting of green tea, fresh tea leaves, grape seed, grape seed coat, cube gambir, red algae and extracts thereof.

The composition containing as its main component proanthocyanidin oligomer to which a substance having a phloroglucinol ring structure or resorcinol ring structure has been bonded, which is obtainable by heating plant materials comprising proanthocyanidin polymer or aqueous extract thereof comprising proanthocyanidin polymer, with a substance having a phloroglucinol ring structure or resorcinol ring structure in an acidic aqueous solution, is obtainable by heating plant material being an extract produced from a combination of fruit from Lychi chinensis and green leaf of Camellia sinensis in an acidic aqueous solution is Oligonol™.

An oral composition for preventing or treating cardiovascular disease or the complications of hypertension may be provided, the composition comprising 10-5000, preferably 50-2500, most preferably 100-300 mg Olea europaea and 10-5000, preferably 50-2500, most preferably 100-300 mg composition containing as its main component proanthocyanidin oligomer represented by structure (I):

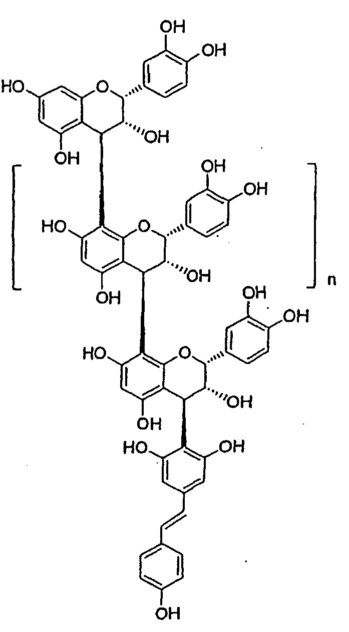

Wherein n is 0 or an integer of 1 or 2.

An oral composition for preventing or treating cardiovascular disease or the complications of hypertension may be provided, the composition comprising 10-5000, preferably 50-2500, most preferably 100-300 mg Olea europaea and 10-5000, preferably 50-2500, most preferably 100-300 mg composition containing as its main component proanthocyanidin oligomer represented by structure (II): 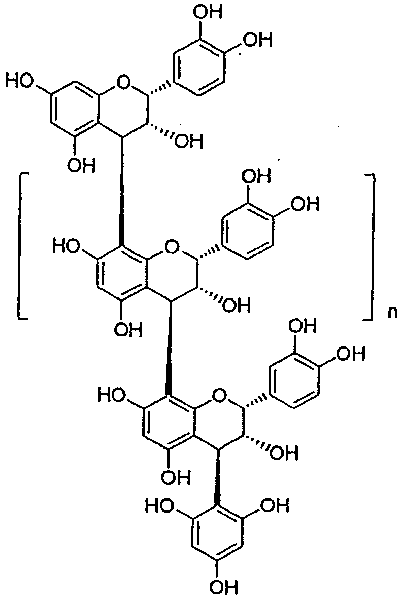 wherein n is 0 or an integer of 1 or 2.

The aqueous ethanol extract of Olea europaea is Aquasol™.

The foregoing compositions may be used in a method for preventing or treating cardiovascular disease or the complications of hypertension in a human being is provided, the method comprising the step of administering to a person in need therefore the composition of any of the first, second or third aspects of the invention.

In a second aspect of the invention, a composition according to the first aspect of the invention for use as a medicament for a human being is provided.

In a third aspect of the invention, a composition according to the first aspects of the invention for preventing or treating cardiovascular disease or the complications of hypertension in a human being is provided.

In a forth aspect of the invention, use of the composition of the first aspect of the invention for the manufacture of a medicament for preventing or treating cardiovascular disease or the complications of hypertension in a human being is provided.

### Summary of the figures

The invention is illustrated with reference to
Figure 1 which shows interleukin 6 (IL-6) response (pg per µg protein) for primary dermal fibroblasts oxidatively stressed with 1 µM phorbol myristate acetate (PMA) subsequently treated with 10 µg/ml Oligonol™, 100 µg/ml Aquasol™ (denoted "Olive" in figure) or both;
Figure 2 which shows interleukin 6 response (pg per µg protein) for primary dermal fibroblasts oxidatively stressed with 1 µM phorbol myristate acetate (PMA) subsequently treated with 0.1, 1 and 10 µg/ml Oligonol™, 100 µg/ml Aquasol™ (denoted "Olive" in figure) or combinations thereof;
Figure 3 which shows interleukin 6 response (pg per µg protein) for primary dermal fibroblasts oxidatively stressed with 1 µM phorbol myristate acetate (PMA) subsequently treated with 0.1, 1 and 10 µg/ml Oligonol™, 100 µM oleuropein or combinations thereof;
Figure 4 which shows interleukin 6 response (pg per µg protein) for primary dermal fibroblasts oxidatively stressed with 1 µM phorbol myristate acetate (PMA) subsequently treated with 10 µg/ml Oligonol™, and 1 and 10 µM tyrosol or combinations thereof;
Figure 5 which shows interleukin 6 response (pg per µg protein) for primary dermal fibroblasts oxidatively stressed with 1 µM phorbol myristate acetate (PMA) subsequently treated with 10 µg/ml Oligonol™, and 0.1, 1 and 10 µM hydroxytyrosol or combinations thereof; and
Figure 6 which shows heme oxygenase 1 (HO-1) (pg per µg protein) for control (untreated human umbilical vein endothelial cells); 2 and 4 µg/ml Oligonol™; 5, 10 and 20 µg/ml Aquasol™ (denoted "Olive" in figure); and combinations thereof.

### Detailed description of the invention

Oral compositions of the invention may be in the form of capsules, pills, tablets, granules, solutions, suspensions or emulsions. The amount of aqueous ethanol extract of Olea europaea or oleuropein, or composition containing as its main component proanthocyanidin oligomer to which a substance having a phloroglucinol ring structure or resorcinol ring structure has been bonded, which is obtainable by heating plant materials comprising proanthocyanidin polymer or extract thereof comprising proanthocyanidin polymer, with a substance having a phloroglucinol ring structure or resorcinol ring structure in an acidic aqueous solution in a capsule, pill or tablet, will not be in excess of the recommended daily limit for an adult person. Preparation of compositions of the invention in the aforementioned oral formats is well known to the person skilled in the art.

### Example 1

### Outline of Experimental Approach

An *in-vitro* model has been developed to investigate the impact of oxidative stress on the inflammatory status of dermal fibroblast cells, in which:
a. Cells are grown in 6-well (9.5cm²) plates.
b. The cells are oxidatively stressed with 1 µM phorbol myristate acetate (PMA).
c. Cell culture supernatant and cell pellets were harvested at 24 hours (t24) post-PMA treatment.
d. All cell culture supernatant was assayed for lactate dehydrogenase (LDH), as a measure of cytotoxicity and Interleukin6 (IL-6) synthesis.

### Culture of Dermal Cells

Primary human dermal fibroblast cells were cultured and passaged in Dulbecco's Modified Eagle Serum (DMEM) (Gibco) supplemented with 10 % foetal bovine serum (FBS). Cells were routinely plated out in 6-well tissue culture dishes, at a seeding density of ~5000 cells/cm² in 2 ml complete medium/well for 24 hours, and incubated at 37°C in 5 % CO₂. Media was removed and cells grown in DMEM and 1 % FBS 24 hours prior to treatments.

### Addition of Test Solutions

Test solutions were prepared in DMEM containing 1 % FBS. Dermal fibroblasts were oxidatively stressed for 24 hours with 1 µM PMA (Sigma P8139) in the presence of the test solutions. The treated supernatant (cell culture supernatant) was removed and stored at -20°C prior to IL-6 and LDH analysis.

The dermal fibroblasts were washed with 500 µl of Dulbecco's phosphated buffer solution (PBS) and 1 ml of trypsin/EDTA solution (Invitrogen) added and plate incubated at 37°C until the cells detached. The cells were then centrifuged immediately at 13000 rpm for 10 minutes and the supernatant discarded. The resulting cell pellet was washed with 500 µl of PBS and centrifuged as before. The supernatant was then discarded and cell pellet stored at -20°C prior to cell lysis (for quantitation of protein).

All cell pellets were lysed on ice for 30 minutes in 1 ml cell lysis buffer per 2.5 x 10⁶ cells. The lysis buffer contained 1% nonyl phenoxypolyethoxylethanol (Tergitol-type NP-40), 0.1% sodium deoxycholate, 0.1% sodium dodecyl sulphate (SDS), 6 mM sodium chloride and 0.05M tris(hydroxymethyl)aminomethane at pH 7.6. Protease inhibitor cocktail (1000X; Sigma P8340) was added prior to use at a level of 10 µl/ml of lysis buffer. The partially lysed cell pellets were completely homogenised with a pellet pestle and unwanted cell debris removed by centrifugation for 20 minutes at 20,000g at 4°C. The clarified cell lysate was frozen at -80°C until needed.

Cell culture supernatent was examined for cytotoxicity using the Promega CytoTox 96 non-radioactive cytotoxicity assay. This assay quantitatively measures lactate dehydrogenase (LDH) released upon cell lysis and is a good indication of cell viability. 50 µl of cell culture supernatent or control medium was added to duplicate wells of a 96 well plate. 50 µl of CytoTox reagent was added and mixed well. The plate was incubated in the dark at room temperature for 30 minutes after which 50 µl of stop solution was added to each well and the absorbance of the plate read at 492 nm. Any test samples giving an absorbance value of more than double that of the control medium was considered to be cytotoxic. No results have been included from samples that showed any signs of cytotoxicity.

The total protein concentration of each clarified cell lysate was measured using the Pierce BCA protein assay kit so that the response to effect of the test substances could be normalised to ug protein. A set of eight standard solutions ranging from 0 to 1200 µg/ml protein was prepared from the supplied 2 mg/ml bovine serum albumin (BSA) stock solution. 10 µl of standard or cell lysate was added to duplicate wells of a flat-bottomed 96-well plate. The reagent solution was prepared according to the kit instructions from 50 parts reagent A and 1 part reagent B. 200 µl of the final reagent was added to each well. The plate was mixed, covered and incubated at 37°C for 30 minutes and absorbance read at 562 nm. A protein standard curve was constructed and used to determine the protein concentration of each cell lysate.

The IL-6 concentration of each cell culture supernatant was assayed using the QuantiGlo Q6000 Human IL-6 assay (R&D Systems) according to the manufacturer's instructions. Six IL-6 standards were prepared in calibrator diluent at concentrations ranging from 0 to 3000 pg/ml. 50 µl of assay diluent and 150 µl of cell culture supernatant or standard was added to duplicate wells. The plate was incubated at room temperature for 2 hours on a horizontal orbital plate shaker (about 500rpm) before being washed four times with wash buffer. 200 µl of IL-6 conjugate was added to each well and the plate incubated at room temperature for 3 hours on a horizontal orbital plate shaker (about 500rpm). The plate was washed as before. Each well received 200 µl of substrate solution and the plate incubated at room temperature for 40 minutes. The relative light unit (RLU) of each well was determined using a luminometer set with 1 minute lag time, 1 second/well read time, summation mode and automatic gain on. A standard curve was plotted of mean RLU versus IL-6 concentration and the line of best fit calculated by regression analysis and the unknown concentration of IL-6 protein in all the samples estimated from this.

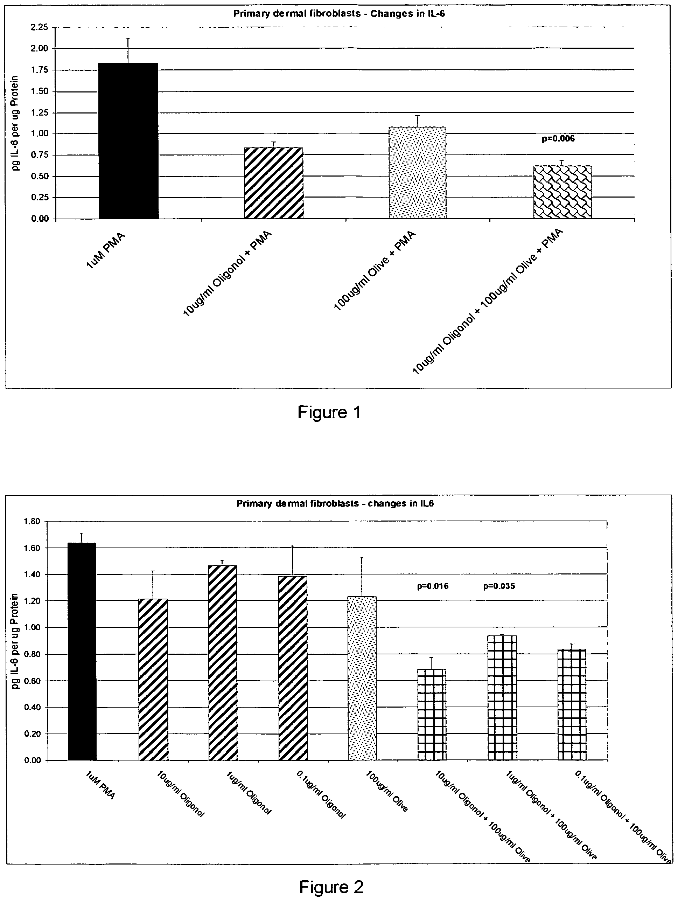

Figure 1 shows IL-6 response (pg per µg protein) for primary dermal fibroblasts oxidatively stressed with 1 µM phorbol myristate acetate (PMA) subsequently treated with 10 µg/ml Oligonol™, 100 µg/ml Aquasol™ or both. Thus when primary dermal fibroblasts were treated with PMA, a known pro-inflammatory agent, the level of IL-6 expressed was significant. On subsequent treatment with Oligonol™ or Aquasol™, a significant reduction in IL-6 expression was noted. However when the primary dermal fibroblasts were subsequently treated with a combination of Oligonol™ and Aquasol™, a significant (p=0.006) synergistic reduction in IL-6 expression was observed. Thus it would be expected that such a combination would be useful for preventing or treating cardiovascular disease or the complications of hypertension in a human being.

As mentioned hereinabove, IL-6 is a pro-inflammatory cytokine and generic marker of inflammation in cardiovascular disease. It is thought that both Oligonol™ and Aquasol™ activate a number of different receptors and inflammatory pathways in a synergistic manner and it would be expected that such a combination would be useful for preventing or treating cardiovascular disease or the complications of hypertension in a human being.

### Example 2

Figure 2 shows IL-6 response (pg per µg protein) for primary dermal fibroblasts oxidatively stressed with 1 µM phorbol myristate acetate (PMA) subsequently treated with 0.1, 1 and 10 µg/ml Oligonol™, 100 µg/ml Aquasol™ or combinations thereof. Thus when primary dermal fibroblasts were treated with PMA, a known pro-inflammatory agent, the level of IL-6 expressed was significant. On subsequent treatment with Oligonol™ or Aquasol™, a slight reduction in IL-6 expression was noted. However when the primary dermal fibroblasts were subsequently treated with combinations of Oligonol™ and Aquasol™, significant (p = 0.16, 0.35 and 0.015) synergistic reductions in IL-6 expression was observed. Thus it would be expected that such a combination would be useful for preventing or treating cardiovascular disease or the complications of hypertension in a human being.

As mentioned hereinabove, IL-6 is a pro-inflammatory cytokine and generic marker of inflammation in cardiovascular disease. It is thought that both Oligonol™ and Aquasol™ activate a number of different receptors and inflammatory pathways in a synergistic manner and it would be expected that such a combination would be useful for preventing or treating cardiovascular disease or the complications of hypertension in a human being.

### Example 3

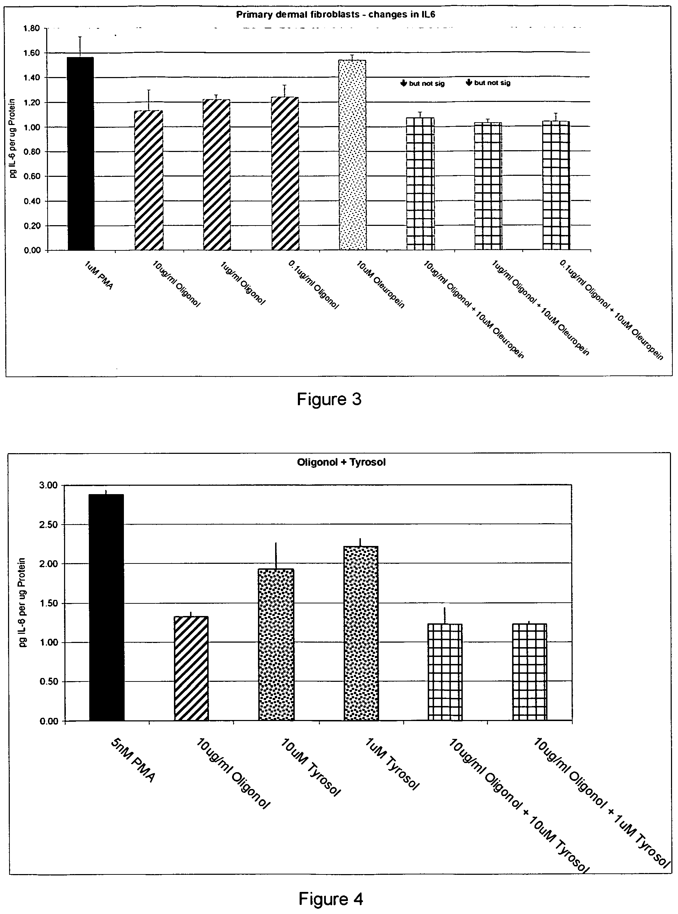

Figure 3 shows IL-6 response (pg per µg protein) for primary dermal fibroblasts oxidatively stressed with 1 µM phorbol myristate acetate (PMA) subsequently treated with 0.1, 1 and 10 µg/ml Oligonol™, 10uM oleuropein or combinations thereof. Thus when primary dermal fibroblasts were treated with PMA, a known pro-inflammatory agent, the level of IL-6 expressed was significant. On subsequent treatment with Oligonol™ or oleuropein, a slight reduction in IL-6 expression was noted. However when the primary dermal fibroblasts were subsequently treated with combinations of Oligonol™ and oleuropein, a significant (p = 0.025) synergistic reduction in IL-6 expression was observed for the combination of 0.1 µg/ml Oligonol™ and 10uM oleuropein although reductions were also seen with combination of 1 µg/ml Oligonol™ and 10uM oleuropein and 10 µg/ml Oligonol™ and 10uM oleuropein. Thus it would be expected that such a combination would be useful for preventing or treating cardiovascular disease or the complications of hypertension in a human being.

As mentioned hereinabove, IL-6 is a pro-inflammatory cytokine and generic marker of inflammation in cardiovascular disease. It is thought that both Oligonol™ and Aquasol™ activate a number of different receptors and inflammatory pathways in a synergistic manner and it would be expected that such a combination would be useful for preventing or treating cardiovascular disease or the complications of hypertension in a human being. As mentioned hereinabove, oleuropein is an important constituent of Aquasol™ and has now been identified as at least partially responsible for the synergistic down-regulation of IL-6 seen when combining Oligonol™ and Aquasol™.

### Example 4

As hydroxyltyrosol is an important constituent of Aquasol™, tyrosol was also tested with Oligonol™. Figure 4 shows IL-6 response (pg per µg protein) for primary dermal fibroblasts oxidatively stressed with 1 µM phorbol myristate acetate (PMA) subsequently treated with 10 µg/ml Oligonol™ and 1 and 10uM tyrosol or combinations thereof. Thus when primary dermal fibroblasts were treated with PMA, a known pro-inflammatory agent, the level of IL-6 expressed was significant. On subsequent treatment with Oligonol™ a reduction in IL-6 expression was noted. However when the primary dermal fibroblasts were subsequently treated with combinations of Oligonol™ and tyrosol, no significant synergistic reduction in IL-6 expression was observed for any of the combinations.

### Example 5

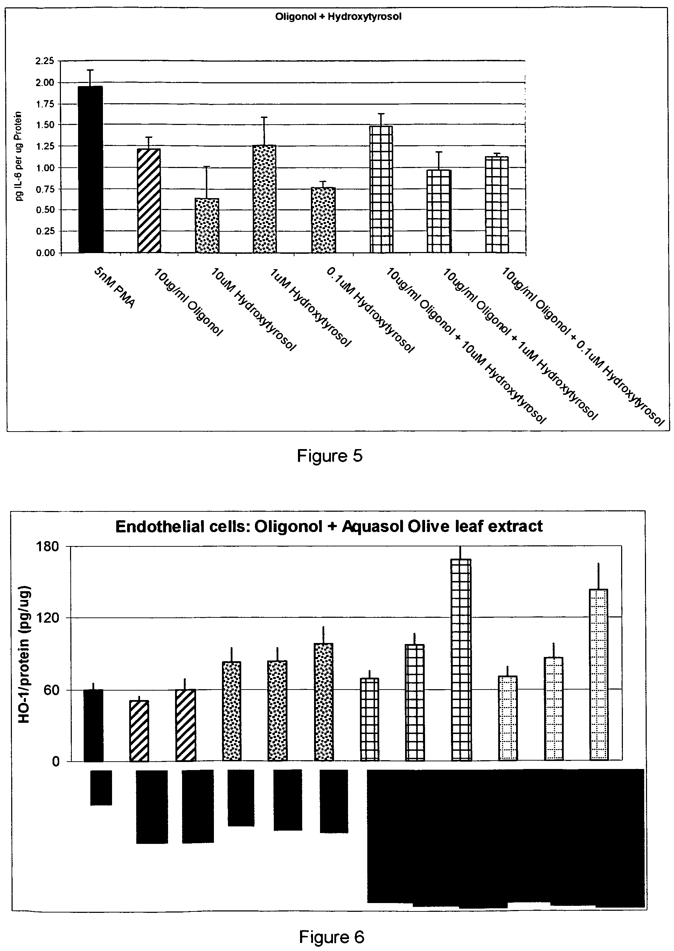

Figure 5 shows IL-6 response (pg per µg protein) for primary dermal fibroblasts oxidatively stressed with 1 µM phorbol myristate acetate (PMA) subsequently treated with 10 µg/ml Oligonol™ and 0.1, 1 and 10uM hydroxytyrosol or combinations thereof. Thus when primary dermal fibroblasts were treated with PMA, a known pro-inflammatory agent, the level of IL-6 expressed was significant. On subsequent treatment with Oligonol™ a reduction in IL-6 expression was noted. However when the primary dermal fibroblasts were subsequently treated with combinations of Oligonol™ and hydroxytyrosol, no significant synergistic reduction in IL-6 expression was observed for any of the combinations.

As mentioned hereinabove, IL-6 is a pro-inflammatory cytokine and generic marker of inflammation in cardiovascular disease. It is thought that both Oligonol™ and Aquasol™ activate a number of different receptors and inflammatory pathways in a synergistic manner and it would be expected that such a combination would be useful for preventing or treating cardiovascular disease or the complications of hypertension in a human being. As mentioned hereinabove, hydroxytyrosol is an important constituent of Aquasol™ and has now been identified as not being responsible, at least on its own, for the synergistic down-regulation of IL-6 seen when combining Oligonol™ and Aquasol™.

### Example 6

Huvec cells, also known as human umbilical vein endothelial cells, (TCS Biologicals) were cultured and passaged in endothelial growth medium (EGM-2) (Biowhittaker) supplemented with heparin, vascular endothelial growth factor (VEGF), gentamicin sulphate, ascorbic acid, human endothelial growth factor (HEGF), hydrocortisone, (human fibroblast growth factor B (HFGF-B), long R insulin-like growth factor 1 (R3-IGF-1) and foetal bovine serum (FBS). Cells were routinely plated out in 6-well tissue culture dishes, at a seeding density of ~5000 cells/cm² in 2 ml complete medium per well 24 hours before starting the experiment and incubated at 37°C in 5% CO₂.

The vascular endothelium, which lines the inner side of blood vessels, is one of the largest secretory tissues of the body. Huvec cells, also known as human umbilical vein endothelial cells, are used as an in-vitro model, as described in Bachettia et al. (Pharmacological Research, 42, 1 (2000)), to examine the potential of different ingredients to help in the prevention and/or therapy of cardiovascular diseases.

The cells were treated with 5 µM 4-hydroxynonenal and aqueous solutions of the test substances for 24 hours and then the level of heme oxygenase 1 measured.

The cells were harvested by adding 1 ml of trypsin/EDTA solution (Invitrogen) and incubated at 37°C until detached. The cells were then centrifuged immediately at 13000 rpm for 10 minutes and the supernatant discarded. The resulting cell pellet was washed with 500 µl of Dulbecco's phosphated buffer solution (PBS) and centrifuged as before. The supernatant was discarded as before and the cell pellet stored at -20°C prior to cell lysis.

All cell pellets were lysed on ice for 30 minutes in 1 ml cell lysis buffer per 2.5 x 10⁶ cells. The lysis buffer contained 1% nonyl phenoxypolyethoxylethanol (Tergitol-type NP-40), 0.1% sodium deoxycholate, 0.1% sodium dodecyl sulphate (SDS), 6 mM sodium chloride and 0.05M tris(hydroxymethyl)aminomethane at pH 7.6. Protease inhibitor cocktail (1000X; Sigma P8340) was added prior to use at a level of 10 µl/ml of lysis buffer. The partially lysed cell pellets were completely homogenised with a pellet pestle and unwanted cell debris removed by centrifugation for 20 minutes at 20,000g at 4°C. The clarified cell lysate was frozen at -80°C until needed.

Cell lysate was examined for cytotoxicity and the total protein concentration of each cell lysate were measured as previously described in example 1.

Huvec cell lysate was diluted in sample diluent, and 100 µl transferred to a precoated anti-human heme oxygenase 1 immunoassay plate (Assay Designs). A 7-point recombinant heme oxygenase 1 standard curve, ranging from 25 to 0.39 ng/ml, was also prepared in sample diluent. The immunoassay plate was incubated at room temperature for 30 minutes, washed six times with wash buffer, and incubated for a further 60 minutes at room temperature with 100 µl/well of anti-heme oxygenase 1 antibody solution. The plate was washed as above and incubated for 30 minutes at room temperature with 100 µl/well of horseradish peroxidise conjugate solution. Again the plate was washed as above and 100 µl/well of tetramethylbenzidine substrate added to each well and stored for 15 minutes at room temperature in the dark. Following the addition of 100 µl/well of stop solution, the absorbance was read at 450 nm and the unknown biopsy levels of heme oxygenase 1 were extrapolated from the standard curve.

Figure 6 shows heme oxygenase 1 (HO-1) (pg per µg protein) for control (untreated human umbilical vein endothelial cells); 2 and 4 µg/ml Oligonol™; 5, 10 and 20 µg/ml Aquasol™ (denoted "Olive" in figure); and combinations thereof. A combination of Oligonol™ and Aquasol™ appears to significantly and synergistically increase heme oxygenase 1 levels.

Murashima et al (Biofactors, 22, 1-4, 271-5 (2004)) describe that nuclear factor erythroid-derived 2 (Nrf2) transcription factors mediate low density lipoprotein (LDL) oxidation. Marco et al (N. Engl. J. Med., 337, 408-416 (1997)) describe how atherosclerosis develops from LDL becoming oxidized by free radicals, particularly reactive oxygen species. Therefore activation of Nrf2 transcription factors should lead to inhibition of oxidation of LDL. Lee et al (Journal of Biochemistry and Molecular Biology, 37, 2, 139-143 (2004)) describe enzymes induced by Nrf2 transcription factors include Heme Oxygenase-1 (HO-1). Thus Heme Oxygenase-1 (HO-1) is an indicator or end marker of Nrf2 activation and hence the synergistic up-regulation of HO-1 on combining Oligonol™ and Aquasol™ would be expected to lead to a synergistic reduction in LDL oxidation indirectly reducing the onset of atherosclerosis.

Heme oxygenase 1 may be activated using a number of different routes (via induction of Nrf2 gene expression and nuclear translocation). Nrf2 transcription factors up-regulate the messenger ribonucleic acid (mRNA), protein and activity for HO-1 and it is believed that both Oligonol™ and Aquasol™ target and activate different parts of the Nrf2 pathway. The results now show that this up-regulation occurs in a synergistic manner, for example, through conformational changes or increases in phosphorylation which may result in increases in nuclear translocation of Nrf2.

## Claims

1. An oral composition for preventing or treating cardiovascular disease or the complications of hypertension, the composition comprising an aqueous ethanol extract of Olea europaea or oleuropein, and a composition containing as its main component proanthocyanidin oligomer to which a substance having a phloroglucinol ring structure or resorcinol ring structure has been bonded, which is obtainable by heating plant materials comprising proanthocyanidin polymer or aqueous extract thereof comprising proanthocyanidin polymer, with a substance having a phloroglucinol ring structure or resorcinol ring structure in an acidic aqueous solution, obtainable by heating plant material being an extract produced from a combination of fruit from Lychi chinensis and green leaf of Camellia sinensis in an acidic aqueous solution is Oligonol™; and
wherein the aqueous ethanol extract of Olea europaea is a 90:10 v/v ethanol:demineralised water extract of the leaves of Olea europaea, the extract comprising 2.5 % w/v oleuropein and 1.9 % w/v hydroxytyrosol.

2. A composition according to claim 1 comprising 10-5000, preferably 50-2500, most preferably 100-300 mg Olea europaea and 10-5000, preferably 50-2500, most preferably 100-300 mg composition containing as its main component proanthocyanidin oligomer to which a substance having a phloroglucinol ring structure or resorcinol ring structure has been bonded, which is obtainable by heating plant materials comprising proanthocyanidin polymer or aqueous extract thereof comprising proanthocyanidin polymer, with a substance having a phloroglucinol ring structure or resorcinol ring structure in an acidic aqueous solution.

3. A composition according to claim 1 or claim 2 wherein the weight ratio of aqueous ethanol extract of Olea europaea to composition containing as its main component proanthocyanidin oligomer to which a substance having a phloroglucinol ring structure or resorcinol ring structure has been bonded, which is obtainable by heating plant materials comprising proanthocyanidin polymer or aqueous extract thereof comprising proanthocyanidin polymer, with a substance having a phloroglucinol ring structure or resorcinol ring structure in an acidic aqueous solution is 10:1 to 1000:1, preferably 10:1 to 100:1.

4. A composition according to any one of claims 1 to 3 for use as a medicament for a human being.

5. A composition according to any one of claims 1 to 3 for preventing or treating cardiovascular disease or the complications of hypertension in a human being.

6. Use of the composition of any one of claims 1 to 3 for the manufacture of a medicament for preventing or treating cardiovascular disease or the complications of hypertension in a human being.

## Patentansprüche

1. Orale Zusammensetzung zur Vorbeugung oder Behandlung von kardiovaskulären Erkrankungen oder von Komplikationen von Bluthochdruck, wobei die Zusammensetzung einen wässrigen Ethanolextrakt von Olea europaea oder Oleuropein umfasst, und eine Zusammensetzung, die als Hauptbestandteil ein Proanthocyanidin-Oligomer enthält, an das eine Substanz mit einer Phloroglucinol-Ringstruktur oder Resorcinol-Ringstruktur gebunden wurde, erhältlich durch Erhitzen von Pflanzenmaterialien, umfassend ein Proanthocyanidin-Polymer oder einen wässrigen Extrakt davon, der ein Proanthocyanidin-Polymer umfasst, mit einer Substanz, die eine Phloroglucinol-Ringstruktur oder Resorcinol-Ringstruktur aufweist, in einer sauren wässrigen Lösung, erhältlich durch Erhitzen von Pflanzenmaterial, das ein Extrakt, hergestellt aus einer Kombination von Früchten des Lychi chinensis und dem grünem Blatt des Camellia sinensis, in einer sauren wässrigen Lösung, ist, und ist Oligonol™; und wobei der wässrige Ethanolextrakt von Olea europaea ein 90:10 v/v Ethanol : demineralisiertes Wasser-Extrakt der Blätter von Olea europaea ist, wobei der Extrakt 2.5% w/v Oleuropein und 1.9% w/v Hydroxytyrosol umfasst.

2. Zusammensetzung gemäß Anspruch 1, umfassend 10-5000, vorzugsweise 50-2500, höchst bevorzugt 100-300 mg Olea europaea und 10-5000, vorzugsweise 50-2500, höchst bevorzugt 100-300 mg einer Zusammensetzung, die als Hauptbestandteil ein Proanthocyanidin-Oligomer enthält, an das eine Substanz mit einer Phloroglucinol-Ringstruktur oder Resorcinol-Ringstruktur gebunden wurde, erhältlich durch Erhitzen von Pflanzenmaterialien, umfassend ein Proanthocyanidin-Polymer oder einen wässrigen Extrakt davon, der ein Proanthocyanidin-Polymer umfasst, mit einer Substanz, die eine Phloroglucinol-Ringstruktur oder Resorcinol-Ringstruktur aufweist, in einer sauren wässrigen Lösung.

3. Zusammensetzung gemäß Anspruch 1 oder Anspruch 2, wobei das Gewichtsverhältnis des wässrigen Ethanolextrakts von Olea europaea zu der Zusammensetzung, die als Hauptbestandteil ein Proanthocyanidin-Oligomer enthält, an das eine Substanz mit einer Phloroglucinol-Ringstruktur oder Resorcinol-Ringstruktur gebunden wurde, erhältlich durch Erhitzen von Pflanzenmaterialien, umfassend ein Proanthocyanidin-Polymer oder einen wässrigen Extrakt davon, der ein Proanthocyanidin-Polymer umfasst, mit einer Substanz, die eine Phloroglucinol-Ringstruktur oder Resorcinol-Ringstruktur aufweist, in einer sauren wässrigen Lösung, 10:1 bis 1000:1, vorzugsweise 10:1 bis 100:1 beträgt.

4. Zusammensetzung gemäß irgendeinem der Ansprüche 1 bis 3 zur Verwendung als Medikament für einen Menschen.

5. Zusammensetzung gemäß irgendeinem der Ansprüche 1 bis 3 zur Vorbeugung oder Behandlung von kardiovaskulären Erkrankungen oder von Komplikationen von Bluthochdruck.

6. Verwendung der Zusammensetzung gemäß irgendeinem der Ansprüche 1 bis 3 zur Herstellung eines Medikaments zur Vorbeugung oder Behandlung von kardiovaskulären Erkrankungen oder von Komplikationen von Bluthochdruck.

## Revendications

1. Composition orale destinée à la prévention ou au traitement d'une maladie cardiovasculaire ou des complications de l'hypertension, la composition comprenant un extrait d'éthanol aqueux d'Olea europaea ou d'oleuropéine, et une composition contenant en tant que composant principal un oligomère de proanthocyanidine auquel une substance présentant une structure cyclique de type phloroglucinol ou une structure cyclique de type résorcinol a été liée, qui peut être obtenue en chauffant des matières végétales comprenant un polymère de proanthocyanidine ou un extrait aqueux de celui-ci comprenant un polymère de proanthocyanidine, avec une substance présentant une structure cyclique de type phloroglucinol ou une structure cyclique de type résorcinol dans une solution aqueuse acide, pouvant être obtenue en chauffant une matière végétale constituée d'un extrait produit à partir d'une combinaison de fruit de Litchi sinensis et de feuilles vertes de Camellia sinensis dans une solution aqueuse acide qui est de l'Oligonol^{™} ; et
dans laquelle l'extrait d'éthanol aqueux d'Olea europaea est un extrait à 90/10 en v/v d'éthanol/eau déminéralisée des feuilles d'Olea europaea, l'extrait comprenant 2,5 % en p/v d'oleuropéine et 1,9 % en p/v d'hydroxytyrosol.

2. Composition selon la revendication 1 comprenant de 10 à 5 000, de préférence de 50 à 2 500, de manière préférée entre toutes de 100 à 300 mg d'Olea europaea et de 10 à 5 000, de préférence de 50 à 2 500, de manière préférée entre toutes de 100 à 300 mg d'une composition contenant en tant que composant principal un oligomère de proanthocyanidine auquel une substance présentant une structure cyclique de type phloroglucinol ou une structure cyclique de type résorcinol a été liée, qui peut être obtenue en chauffant des matières végétales comprenant un polymère de proanthocyanidine ou un extrait aqueux de celui-ci comprenant un polymère de proanthocyanidine, avec une substance présentant une structure cyclique de type phloroglucinol ou une structure cyclique de type résorcinol dans une solution aqueuse acide.

3. Composition selon la revendication 1 ou la revendication 2 dans laquelle le rapport pondéral de l'extrait d'éthanol aqueux d'Olea europaea à la composition contenant en tant que composant principal un oligomère de proanthocyanidine auquel une substance présentant une structure cyclique de type phloroglucinol ou une structure cyclique de type résorcinol a été liée, qui peut être obtenue en chauffant des matières végétales comprenant un polymère de proanthocyanidine ou un extrait aqueux de celui-ci comprenant un polymère de proanthocyanidine, avec une substance présentant une structure cyclique de type phloroglucinol ou une structure cyclique de type résorcinol dans une solution aqueuse acide est de 10/1 à 1 000/1, de préférence de 10/1 à 100/1.

4. Composition selon l'une quelconque des revendications 1 à 3 pour utilisation en tant que médicament pour un être humain.

5. Composition selon l'une quelconque des revendications 1 à 3 destinée à la prévention ou au traitement d'une maladie cardiovasculaire ou des complications de l'hypertension chez un être humain.

6. Utilisation de la composition selon l'une quelconque des revendications 1 à 3 pour la fabrication d'un médicament destiné à la prévention ou au traitement d'une maladie cardiovasculaire ou des complications de l'hypertension chez un être humain.
